# EUROPEAN PATENT APPLICATION

(11) **EP 4 686 716 A1**
(43) Date of publication of application: **04.02.2026**
(21) Application number: 24191704.6
(22) Date of filing: 30.07.2024
(51) Int. Cl.: C07C 29/141, C07C 45/50, C01B 3/00

(54) **ONE-POT CATALYTIC PROCESS FOR THE PREPARATION OF ALCOHOLS USING METHANOL AS SOURCE FOR CARBON MONOXIDE AND HYDROGEN**

(71) Applicant: Max-Planck-Gesellschaft zur Förderung der Wissenschaften e.V., 80539 München (DE)
(72) Inventor: Leitner, Walter, 45470 Mülheim an der Ruhr (DE); Vorholt, Andreas, 45470 Mülheim an der Ruhr (DE); Stahl, Sebastian, 45470 Mülheim an der Ruhr (DE); Vossen, Jeroen, 45470 Mülheim an der Ruhr (DE); Popp, Stephan, 45470 Mülheim an der Ruhr (DE)
(74) Representative: Hoffmann Eitle

(57) **Abstract**

A catalytic one-pot process for the preparation of an alcohol is provided. The process comprises decomposition of methanol into synthesis gas, the hydroformylation of an optionally substituted alkene using said synthesis gas and hydrogenation to the corresponding alcohol. The catalyst system combines a hydroformylation catalyst and a transition metal complex catalyst.

## Description

### Field of the Invention

The present invention is directed towards a catalytic process for the preparation of an alcohol. In particular, the process comprises the decomposition of methanol to synthesis gas which is used for the hydroformylation of an alkene to an aldehyde, and hydrogenation to the corresponding alcohol.

### Background of the Invention

Hydroformylation of alkenes is one of the most important chemical reactions and produces more than 10 million tons of aldehydes per year. The possible side reactions are hydrogenation and isomerization, and the corresponding byproducts are alkanes and internal olefins. Several transition metals such as Ir, Rh, Co, Ru, Fe and Pd have been used to catalyze this reaction, but only Co and Rh are used on an industrial scale due to their high activity and selectivity. Suitable catalysts include Rh/phosphine and Rh/phosphite catalysts which are prepared from the precursor Rh(acac)(CO)₂.

Methanol is widely inferred as a potential substitute for fossil fuels and a crucial molecular pivot at the interface of the energy and chemical sectors because it can be produced from renewable resources, including direct hydrogenation of carbon dioxide. It is easily stored, transported, and distributed in liquid form.

For use in the chemical sector, methanol may be decomposed by catalytic dehydrogenation to a mixture of hydrogen and carbon monoxide in a molar ratio of 2:1 which is known as "synthesis gas". Typically, the reaction is carried out in the gas phase using heterogenous catalysts at temperatures around or above 200°C. However, some homogenous catalysts have also been described. For example, A. Kaithal, B. Chatterjee, C. Werlé, W. Leitner, Angew. Chem. Int. Ed. 2021, 60, 26500-26505 discloses Ru, Ir and Mn complexes for homogenous catalysis.

WO 2020/136003 A1 and DE 10 2021 006 375 A1 disclose processes for producing methanol by catalyzed reaction of CO and H₂, wherein the catalyst is a complex comprising a transition metal selected from Mn, Fe and Mo and a pincer type ligand.

So far, methanol has not been used as the source of synthesis gas for hydroformylation of alkenes. However, it would be desirable to provide a process wherein decomposition of methanol can be combined with the preparation of an alcohol by hydroformylation of an alkene and subsequent hydrogenation of the aldehyde, preferably as a one-pot reaction.

### Summary of the Invention

This object is solved by the process according to claim 1. Preferred embodiments are the subject of the dependent claims.

### Brief Description of the Figures

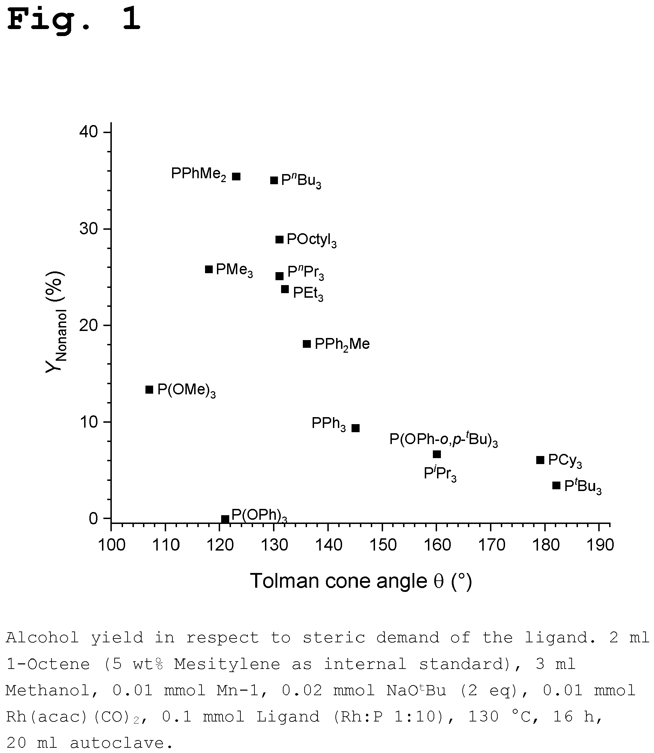

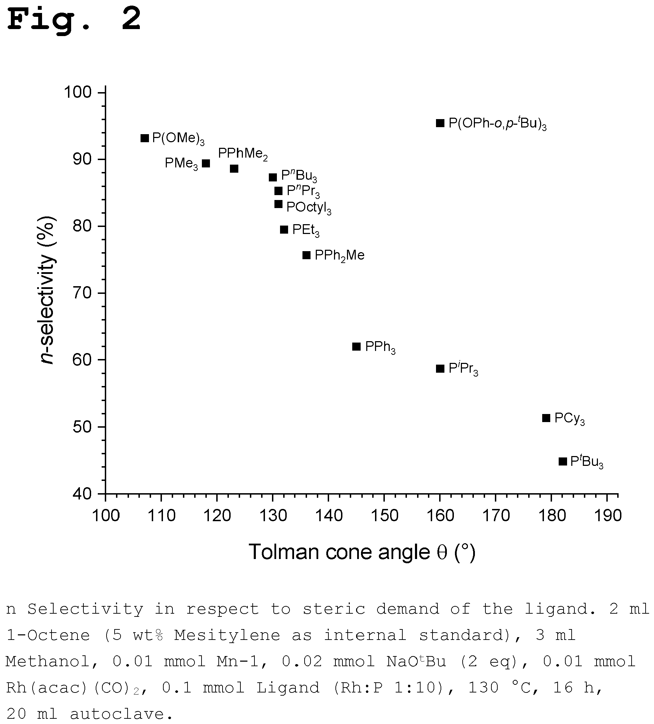

Figs. 1 and 2 show the yield and selectivity of a process according to the invention wherein the transition metal catalyst Mn-1 is combined with various hydroformylation catalysts.

### Detailed Description

The present invention provides a one-pot catalytic process for preparing alcohols which uses methanol as the source of carbon monoxide and hydrogen. This process comprises (1) decomposition of methanol to synthesis gas, (2) hydroformylation of an alkene, and (3) hydrogenation to the corresponding alcohol.

The following scheme depicts the process according to the present invention wherein the alkene is a 1-alkene.

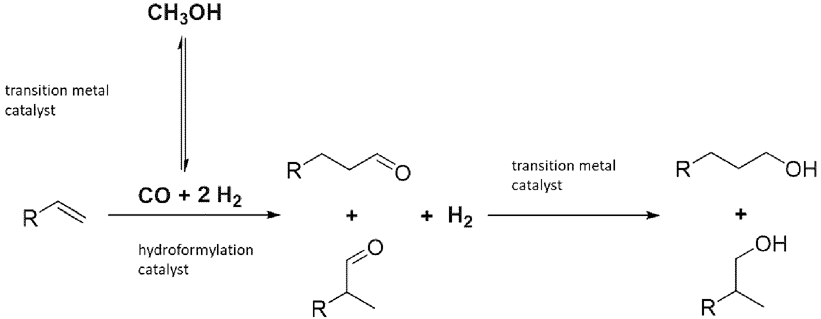

As used herein, the term "one-pot process" refers to a process wherein steps (1), (2) and (3) are carried out in a single vessel / reactor. Because the process of the invention is a one-pot-synthesis, it is associated with low costs and provides the alkanol in a high yield.

When attempting to combine (1) decomposition of methanol with (2) hydroformylation of an alkene and (3) subsequent hydrogenation of the resulting aldehyde, the present inventors surprisingly found that there is little, or even no, ligand exchange between the two catalysts so that they remain active.

In the one-pot process according to the invention, methanol decomposition proceeds without reaching an equilibrium stage because hydroformylation and hydrogenation consume hydrogen and carbon monoxide in a molar ratio of 2:1. In addition, the accumulation of gas(es) is avoided so that the process can be conducted at low pressure which is advantageous over conventional high-pressure processes because simpler equipment can be used.

The process of the present invention provides the alcohol with high selectivity. It is believed that activity of the transition metal catalyst is enhanced because hydrogen which is required for the formation of the hydrogenation active hydrogen complex is provided by decomposition of methanol rather than by gaseous hydrogen.

If 1-alkenes are subjected to the process of the present invention, the corresponding n-alcohols are obtained with high selectivity. It is believed that this selectivity results from the low pressure and low loading of the hydroformylation catalyst.

The catalyst for the process according to the invention comprises (a) a hydroformylation catalyst and (b) a transition metal catalyst. As shown in the scheme above, the transition metal catalyst catalyzes steps (1) and (3), whereas step (2) is catalyzed by the hydroformylation catalyst.

The hydroformylation catalyst to be used in the present invention contains a central atom which is selected from Rh, Co, Ir, Ru, Os, Pt, Pd, Fe and Ni, and one or more ligands which are independently selected from amine (NR₃), phosphine (PR₃), phosphinite (PR₂(OR)), phosphonite (PR(OR)₂), phosphite (P(OR)₃), aminophosphine (PR₂(NR₂)), diaminophosphine (PR(NR₂)₂), triaminophosphine (P(NR₂)₃), phosphoroamidite (P(OR)₂(NR₂)), phosphorodiamidite (P(OR)(NR₂)₂) and halogenophosphine(PX₃). Preferably, each X is independently selected from F, Cl, OR, NR₂ and R, and each R is independently selected from C5-C14 aryl, C1-C10 alkyl and C5-C6 cycloalkyl, preferably Ph and C1-C8 alkyl.

In a particularly preferred embodiment of the present invention, the hydroformylation catalyst is selected from Rh/triphenylphosphine, Rh/triphenylphosphite, Rh/BiPhePhos, and Rh/P(Ph)ₐ(alkyl)_{b}(O-alkyl)_{c}(O-Ph)_{d} wherein a, b, c and d each represent an integer of 0 to 3, a + b + c + d = 3, and the phenyl group in (O-Ph) may optionally be substituted by one or more C1-C6 alkyl. Rh/P(OMe)₃, Rh/PMe₃, Rh/PPhMe₂, Rh/P(ⁿBu)₃, Rh/P(ⁿPr)₃, Rh/P(ⁿOctyl)₃, Rh/PEt₃, Rh/PPh₂Me, Rh/PPh₃, Rh/P(^{t}Bu)₃, Rh/P(cyclohexyl)₃ and Rh/P(O-Ph-o,p-^{t}Bu)₃ are especially preferred.

As used herein, "BiPhePhos" refers to the following ligand:

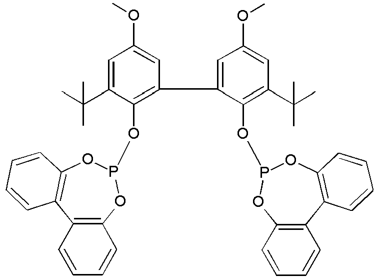

The transition metal complex catalyst to be used in the present invention contains a central ion which is selected from the group comprising Mn, Fe, Cr, Mo, W, Re, Ru, Co, Rh, Ir, Ni and Pd, and Mo, and a pincer type ligand. Preferably, the central ion is Ru, Fe, Ir or Mn, more preferably Ru, Fe or Mn, even more preferably Mn.

As used herein, "pincer type ligand" refers to a tridentate ligand which binds to the central atom via N, P, O and/or S atoms. Preferably, the pincer ligand is a PNP-type pincer ligand, a PNN-type pincer ligand or an NNN-type pincer ligand, more preferably the pincer ligand is a PNP-type pincer ligand or a PNN-type pincer ligand.

In a particularly preferred embodiment of the present invention, the transition metal complex has the formula (1) 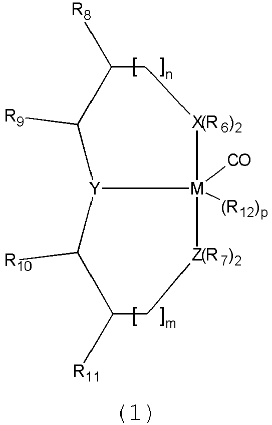 wherein:
M is a Ru, Fe, Mn, or Ir; preferably Ru, Fe or Mn; more preferably Ru or Mn; even more preferably Mn;
each X, Y and Z are independently P, N, NH, O or S;
preferably P or N; more preferably X and Z are P and Y is N; each R₆ and R₇ are independently H, Ph, C5-C10 cycloalkyl or C1-C6 alkyl; preferably Ph, ⁿOctyl, cyclohexyl, ⁱPr, ^{t}Bu, Et or Me;
each R₈, R₉, R₁₀ and R₁₁ are independently H, C1-C10 alkyl, or C5-C10 aryl;
each m and n are independently 0, 1, 2 or 3; preferably 0 or 1; more preferably 0;
each R₁₂ are independently H, CO, Br or Cl;
p is 0, 1 or 2; and
wherein optionally:
   the carbon attached to R₈ forms a double bond with the carbon attached to R₉; and/or
   the carbon attached to R₁₀ forms a double bond with the carbon attached to R₁₁; and/or
   two or more of R₈, R₉, R₁₀, and R₁₁ form a ring system, wherein the ring system is preferably an aromatic C3-C6 monocyclic ring system, a heteroaromatic C3-C6 monocyclic ring system, or an aromatic C9-C14 tricyclic ring system; and/or the carbon attached to R₉ forms a double bond with Y; and/or the carbon attached to R₁₀ forms a double bond with Y.

In a particularly preferred embodiment, X(R₆)₂ and Z(R₇)₂ are independently -PPh₂, -P(C5-C10 cycloalkyl)₂ or -P(C1-C6 alkyl)₂; especially -PPh₂, -P(cyclohexyl)₂, -P(ⁿOctyl)₂, -P(ⁱPr)₂, -P(^{t}Bu)₂, -PEt₂ or -PMe₂.

As used herein, "Ph" refers to a phenyl group, "ⁿOctyl" and "Octyl" refer to an unbranched octyl group, "ⁱPr" refers to an iso-propyl group, "^{t}Bu" refers to a tert-butyl group, "Et" refers to an ethyl group and "Me" refers to a methyl group.

Particular preferred transition metal catalysts are 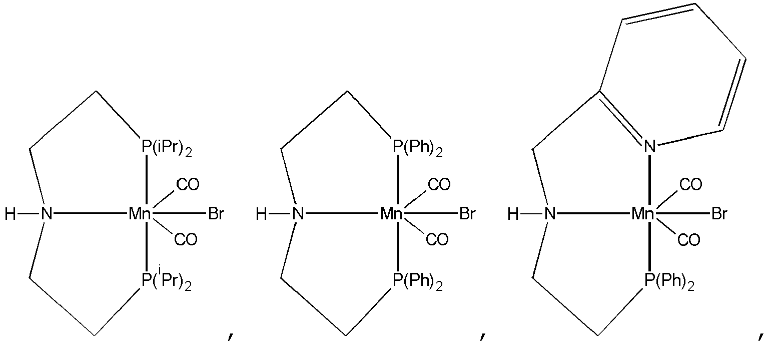 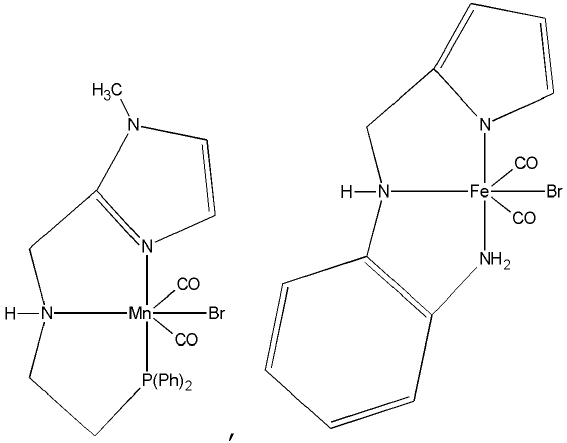 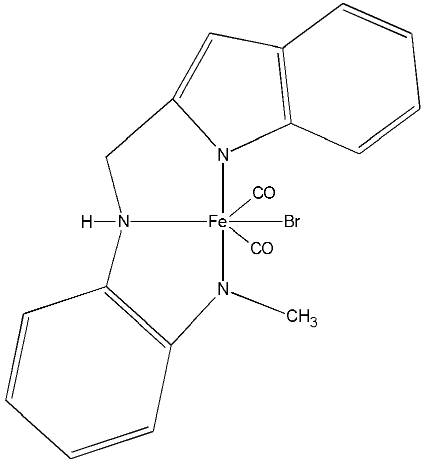 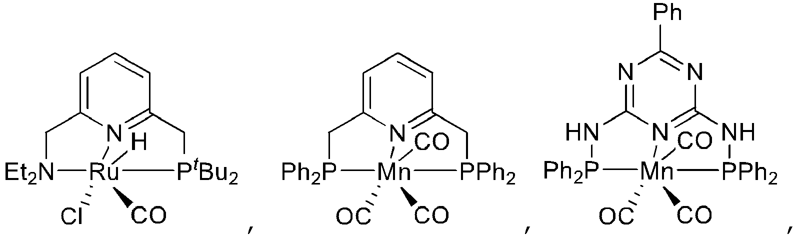 and

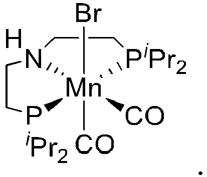

In another preferred embodiment, the transition metal catalyst is Ru/MACHO or Ru/MACHO-BH, preferably Ru/MACHO-BH having the formula

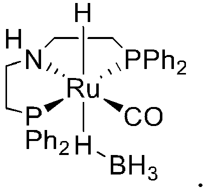

In order to optimize their activity and their stability, the two catalysts are preferably used in a ratio which matches their activity (i.e., the conversion rate obtained by the respective catalyst), thereby reducing / preventing the accumulation of carbon monoxide and/or hydrogen.

The alkene to be converted by the process of the present invention may be a straight or branched 1-alkene or a cycloalkene. The 1-alkene and the cycloalkene may be substituted and may contain one or more additional double bond(s). Suitable substituents of the 1-alkene and the cycloalkene include C1-C10 alkyl, C5-C6 aryl, halogen, -OH, -COOH or COOR, wherein R is C1-C6 alkyl. In a particularly preferred embodiment of the present invention, the alkene is selected from C5-C8 cycloalkene and straight or branched C2-C20 1-alkene which may each include further double bond(s) and may each optionally be substituted with one or more of phenyl, halogen and -OH; more preferably the alkene is selected from styrene, C5-C8 cycloalkene and straight or branched C2-C20 1-alkene, wherein C5-C8 cycloalkene and C2-C20 1-alkene may include further double bond(s); even more preferably the alkene is selected from styrene, ethene, 1-butene, 1-hexene, 1-octene, 1-decene, cyclohexene, 1,5-hexadiene, 1,7-octadien, 7-methyl-3-methylene-1,6-octadiene, 8-brom-1-octene, 5-hexen-1-ol.

In a preferred embodiment according to the present invention, the process is a homogeneous catalytic process.

In a preferred embodiment, the process takes place under inert atmosphere.

The process according to the present invention is preferably conducted at at a temperature of 100°C to 160 C; more preferably 120°C to 150°C, even more preferably 125°C to 135°C.

In a preferred embodiment of the present invention, the reaction conditions are adjusted in a such a manner that the pressure in the reaction vessel does not exceed 10 bar. More preferably, the process is conducted at a pressure of 1 to 10 bar, even more preferably 5 to 10 bar, particularly 7 to 10 bar.

The process according to the invention can be carried out in the presence of one or more solvents. A preferred solvent is toluene.

The process according to the present invention is preferably carried out in the presence of a base; more preferably in the presence of a base which is selected from alcoholates, phosphates, sulfates, carbonates and hydroxides of Li, Na, K or Ca; even more preferably the base is NaO^{t}Bu.

The process according to the invention can be carried out in any means suitable, preferably in an autoclave reactor or a microwave. Suitable reactor types can be selected by the skilled person based on knowledge about the kinetics of the reactions.

### Examples

Hereinafter examples according to the present invention will be presented.

### Example 1

Stock solutions containing transition metal complex (Mn-I) and various Rh catalysts were prepared in an argon-filled glovebox as follows:

Transition metal complex (Mn-I) (0.01 mmol) and NaO^{t}Bu (0.02 mmol, 2eq) were measured into a Schlenk tube and dissolved in 3 ml of methanol.

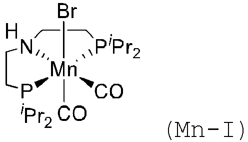

Rh(acac) (CO)₂ (0.01 mmol) and the respective phosphine or phosphite ligand (0.1 mmol) which form the hydroformylation catalyst were measured into a separate Schlenk tube and dissolved in a stock solution of 1-octene with 5%wt of mesitylene (as an internal standard).

A 20 ml stainless steel autoclave was equipped with a stirring bar, closed and evacuated and purged with argon three times. Both stock solutions were added at room temperature through a valve under argon. The autoclave was heated to 130°C for 16 h. Subsequently, the autoclave was cooled down to room temperature and excess pressures was slowly vented. The reaction mixture was analyzed using GC chromatography.

Selectivity and yield of 1-nonanol for the various hydroformylation catalysts are shown in Figs. 1 and 2.

### Examples 2 and 3

Catalyst were prepared as stock solutions in an argon-filled glovebox. Transition metal complex (Mn_I) (0.01 mmol) and NaO^{t}Bu (0.1 mmol, 10 eq) were measured into a Schlenk tube and dissolved in 3 ml of methanol. The components for the hydroformylation catalyst (0.00075 mmol Rh(acac)(CO)₂, 0.1 mmol P(ⁿOctyl)₃) were measured into a separate Schlenk tube and dissolved in 1 or 2 mol of a stock solution of 1-octene with 5%wt of mesitylene (as an internal standard).

A 20 ml stainless steel autoclave was equipped with a stirring bar, closed and evacuated and purged with argon three times. Both stock solutions were added at room temperature through a valve under argon. The autoclave was heated to 130°C for 16 or 72 h. After this, the autoclave was cooled down to room temperature and excess pressures was slowly vented.

The reaction mixture was analyzed using GC chromatography.

| Ex. | 1-Octene (ml) | MeOH (ml) | t (h) | TON_{Hydrof}. | Y_{Nonanol} (%) |
|---|---|---|---|---|---|
| 2 | 2 | 3 | 16 | 7253 | 44.5 |
| 3 | 1 | 3 | 72 | 5284 | 65.0 |

In these experiments, n-selectivity was >90%, and the alcohol selectivity >99.5%.

### Example 4

Catalysts were prepared as stock solutions in an argon-filled glovebox. Transition metal complex (Mn-I) (0.01 mmol) and NaO^{t}Bu (0.02 mmol, 2eq) were measured into a Schlenk tube and dissolved in 3 ml of methanol. The components of the hydroformylation catalyst (0.01 mmol Rh(acac)(CO)₂, 0.1 mmol P(ⁿOctyl)₃) were measured into a separate Schlenk tube and dissolved in a stock solution of 1-hexene with 5%wt of mesitylene (as an internal standard).

A 20 ml stainless steel autoclave was equipped with a stirring bar, closed and evacuated and purged with argon three times. Both stock solutions were added at room temperature through a valve under argon. The autoclave was heated to 130°C for 16 h. After this, the autoclave was cooled down to room temperature and excess pressures was slowly vented.

The reaction mixture was analyzed using GC chromatography. The reaction yielded 3.9% heptanol with a alcohol selectivity >99% and n-selectivity of 72%.

### Example 5

Catalysts were prepared as stock solutions in an argon-filled glovebox. Transition metal complex Ru/MACHO-BH (0.01 mmol) was measured into a Schlenk tube and dissolved in 3 ml of methanol. 0.003 mmol Rh(acac)(CO)₂ and 0.1 mmol P(ⁿOctyl)₃) were measured into a separate Schlenk tube and dissolved in a stock solution of 1-octene with 5%wt of mesitylene.

A 20 ml stainless steel autoclave was equipped with a stirring bar, closed and evacuated and purged with argon three times. Both solutions were added at room temperature through a valve under argon. The autoclave was heated to 130°C for 16 h. After this, the autoclave was cooled down to room temperature and excess pressures was slowly vented.

The reaction mixture was analyzed using GC chromatography. The reaction yielded 4.7% heptanol with a alcohol selectivity 98% and n-selectivity of 86%.

## Claims

1. A catalytic process for the preparation of an alcohol, which comprises (1) decomposition of methanol into synthesis gas, (2) hydroformylation of an optionally substituted alkene using said synthesis gas, and (3) hydrogenation to the corresponding alcohol, **characterized in that** the process is a one-pot process, and that the catalyst comprises:
a) a hydroformylation catalyst, wherein the hydroformylation catalyst contains a central atom which is selected from Rh, Co, Ir, Ru, Os, Pt, Pd, Fe and Ni, and one or more ligands which are independently selected from phosphine, phosphinite, phosphonite, phosphite, aminophosphine, diaminophosphine, triaminophosphine, phosphoroamidite, phosphorodiamidite and halogenophosphine;
b) a transition metal complex catalyst, wherein the transition metal complex contains a central ion which is selected from the group comprising Mn, Fe, Cr, Mo, W, Re, Ru, Co, Rh, Ir, Ni and Pd, and Mo, and a pincer type ligand.

2. The process according to claim 1, wherein transition metal complex catalyst contains a central ion which is selected from Ru, Fe, Ir and Mn, preferably the central ion is Ru, Fe or Mn, more preferably Mn.

3. The process according to claim 1 or 2, wherein the transition metal complex has the formula (1) 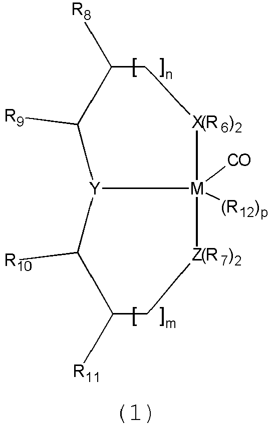 wherein:
M is a Ru, Fe, Mn, or Ir; preferably Ru, Fe or Mn; more preferably Ru or Mn; even more preferably Mn;
each X, Y and Z are independently P, N, NH, O or S; preferably P or N; more preferably X and Z are P and Y is N;
each R₆ and R₇ are independently H, Ph, C5-C10 cycloalkyl or C1-C6 alkyl; preferably Ph, octyl, cyclohexyl, ⁱPr, ^{t}Bu, Et or Me;
each R₈, R₉, R₁₀ and R₁₁ are independently H, C1-C10 alkyl, or C5-C10 aryl;
each m and n are independently 0, 1, 2 or 3; preferably 0 or 1; more preferably 0;
each R₁₂ are independently H, CO, Br or Cl;
p is 0, 1 or 2; and
wherein optionally:
the carbon attached to R₈ forms a double bond with the carbon attached to R₉; and/or
the carbon attached to R₁₀ forms a double bond with the carbon attached to R₁₁; and/or
two or more of R₈, R₉, R₁₀, and R₁₁ form a ring system,
wherein the ring system is preferably an aromatic C3-C6 monocyclic ring system, a heteroaromatic C3-C6 monocyclic ring system, or an aromatic C9-C14 tricyclic ring system; and/or
the carbon attached to R₉ forms a double bond with Y; and/or
the carbon attached to R₁₀ forms a double bond with Y.

4. The process according to claim 3, wherein
X(R₆)₂ and Z(R₇)₂ are independently -PPh₂, -P(C5-C10 cycloalkyl)₂ or -P(C1-C6 alkyl)₂;
preferably -PPh₂, -P(cyclohexyl)₂, -P(octyl)₂, -P(ⁱPr)₂, -P(^{t}Bu)₂, -PEt₂ or -PMe₂.

5. The process according to claim 1 or 2, wherein the transition metal complex is selected from 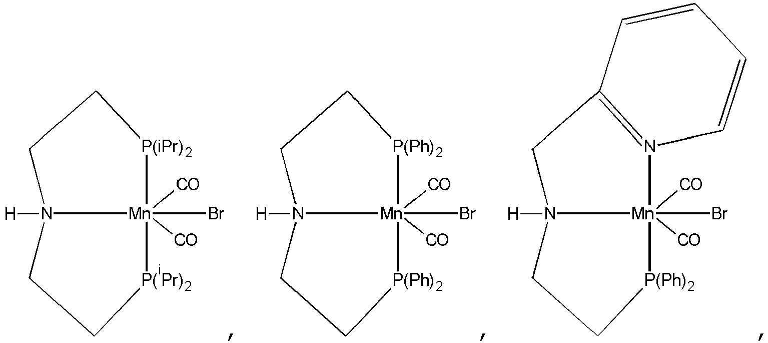 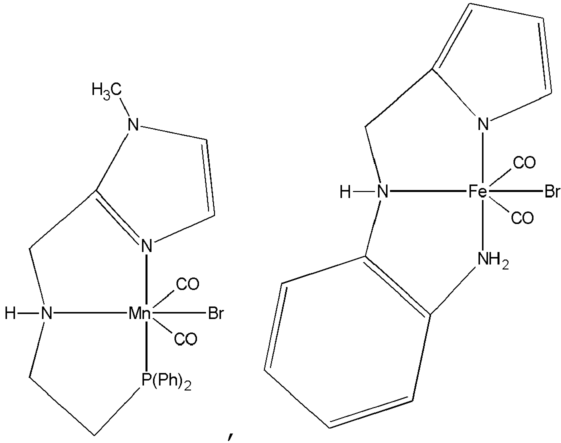 or

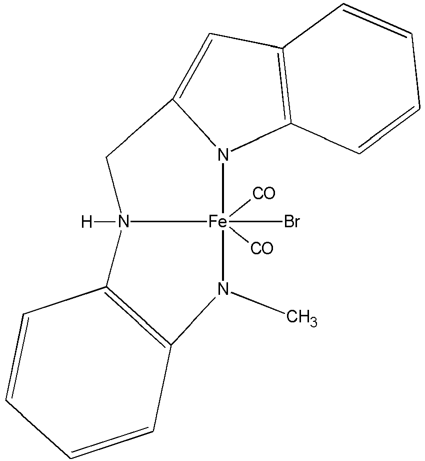

6. The process according to claim 1 or 2, wherein the transition metal complex is selected from

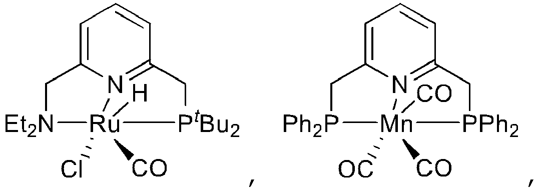

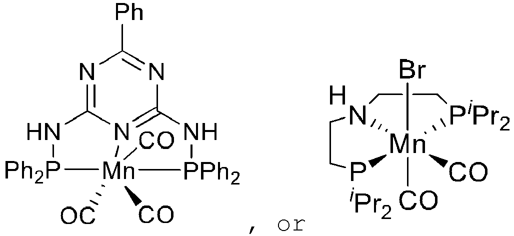

7. The process according to claim 1 or 2, wherein the transition metal complex is selected from Ru-MACHO and Ru-MACHO-BH.

8. The process according to any one of the preceding claims, wherein the process is a homogenous process.

9. The process according to any one of the preceding claims, wherein the alkene is selected from C5-C8 cycloalkene and straight or branched C2-C20 1-alkene which may each include further double bond(s) and may each optionally be substituted with C1-C10 alkyl, C5-C6 aryl, halogen, -OH, -COOH or COOR, wherein R is C1-C6 alkyl; preferably the alkene is selected from C5-C8 cycloalkene and straight or branched C2-C20 1-alkene which may each include further double bond(s) and may each optionally be substituted with one or more of phenyl, halogen and -OH; more preferably the alkene is selected from styrene, C5-C8 cycloalkene and straight or branched C2-C20 1-alkene, wherein C5-C8 cycloalkene and C2-C20 1-alkene may include further double bond(s); even more preferably the alkene is selected from styrene, ethene, 1-butene, 1-hexene, 1-octene, 1-decene, cyclohexene, 1,5-hexadiene, 1,7-octadien, 7-methyl-3-methylene-1,6-octadiene, 8-brom-1-octene, 5-hexen-1-ol.

10. The process according to any one of the preceding claims, wherein the hydroformylation catalyst is selected from Rh/triphenylphosphine, Rh/triphenylphosphite, Rh/BiPhePhos, and Rh/P(Ph)ₐ(alkyl)_{b}(O-alkyl)_{c}(O-Ph)_{d} wherein a, b, c and d each represent an integer of 0 to 3, a + b + c + d = 3, and the phenyl group in (O-Ph) may optionally be substituted by one or more C1-C6 alkyl.

11. The process according to claim 10, wherein the hydroformylation catalyst is selected from Rh/P(OMe)₃, Rh/PMe₃, Rh/PPhMe₂, Rh/P(ⁿBu)₃, Rh/P(ⁿPr)₃, Rh/P(octyl)₃, Rh/PEt₃, Rh/PPh₂Me, Rh/PPh₃, Rh/P(^{t}Bu)₃, Rh/P(cyclohexyl)₃ and Rh/P(O-Ph-o,p-^{t}Bu)₃.

12. The process according to any one of the preceding claims, wherein the process is conducted at a temperature of 100°C to 160 C; preferably 120°C to 150°C, more preferably 125°C to 135°C.

13. The process according to any one of the preceding claims, wherein the process is conducted at a pressure of 1 to 10 bar, preferably 5 to 10 bar, more preferably 7 to 10 bar.

14. The process according to any one of the preceding claims, wherein the process is carried out in the presence of a base: preferably in the presence of a base which is selected from alcoholates, phosphates, sulfates, carbonates and hydroxides of Li, Na, K or Ca; more preferably NaO^{t}Bu.
